# EUROPEAN PATENT APPLICATION

(11) **EP 1 825 881 A1**
(43) Date of publication of application: **29.08.2007**
(21) Application number: 06015667.6
(22) Date of filing: 27.07.2006
(51) Int. Cl.: A61N 5/00

(54) **Simulating the distribution of therapeutic or diagnostic agents in a subject**

(71) Applicant: BrainLAB AG, 85622 Feldkirchen (DE)
(72) Inventor: Rodriguez-Ponce, Maria Inmaculada, Dr., 81669 München (DE); Pedain, Christoph, Dr., 81667 München (DE)
(74) Representative: Schwabe - Sandmair - Marx

(57) **Abstract**

The invention concerns a method for simulating the distribution of at least an agent in a subject, wherein the at least one agent is administered to the subject, using subject-specific information, information about the administration of the at least one agent and agent-specific information, which are combined to calculate the effect of the at least one agent and regions which can be only in a volume of interest, where the agent has an effect in the subject.

## Description

This invention relates to the display of information regarding the therapeutic or diagnostic effects. More specifically, the present invention relates to a method and device for improving the simulation and display of the spatial distribution of a therapeutic and/or diagnostic agent in biological tissue, preferably in combination with information about the efficiency or effect of the agent on the targeted tissue.

US 5,995,864 discloses a system for displaying a model of isodoses used in boron neutron capture therapy (BNCT) which enables a graphic display of an image of a desired anatomical feature of a patient with isodose contours superimposed thereon to reflect radiotherapy treatment.

It is an object of the present invention to enable the planner to see what effect the treatment of a tissue with a therapeutic or diagnostic agent has.

This object is achieved by the method and device as defined in the independent claims. Preferred embodiments are defined in the dependent claims.

According to the inventive method the distribution of at least an agent in tissue is simulated or calculated, wherein a patient or subject-specific information is used together with an agent specific information to obtain from the combination of these pieces of information a combined information which allows to calculate regions within the patient or subject, where the agent which is administered to the subject and for example infused or injected into the patient or subject, has an effect in the patient or subject, e.g. an effect in a specific tissue. Additionally information about the administration is used for calculating the effect or rate or degree of the effect. The patient or subject-specific information can be information about physiology, anatomy, vascular permeability, hydraulic conductivity, diffusivity and/or any other information which can be used to describe the structure or properties of the structure of the subject or patient. The agent specific information is for example any information which describes the effect or behaviour of an agent, such as a therapeutic and/or diagnostic substance and/or a carrier substance thereof, which could be information about the metabolization, loss, influx and/or efflux of the agent or substance. Using the patient specific information together with the agent specific information it can be calculated which effect or effects this agent or substance has in the patient when it is known where and how this agent is brought into the patient. The effect of the substance on the patient or tissue can include binding, necrosis, swelling, uptake, metabolic alterations, genetic alterations, physical alterations, chemical alterations, electrical alterations, morphologic alterations, mutations, physiological alterations or any kind of change of the internal structure of the patient or effects on the tissue of the patient.

Thus, the method according to the present invention can be based on patient-specific information together with information about the properties of the administered agent, such as metabolic tissue-dependent properties, molecule degradation and/or other transport properties and can also take into account physiological tissue properties, like vascular permeability, hydraulic conductivity, pore fraction and diffusivity.

The invention provides thus the possibility that the user gets a prediction of the potential success of the treatment in terms not only of the area covered, but also in terms of the local therapeutic and/or diagnostic effects and in a preferred embodiment also of the degree or distribution of the effect(s).

Thus, the distribution of the administered agent can be simulated and can be displayed with regard to the physiological impact on the covered tissue. The invention considers not only the covered target area, but also the therapeutic and/or diagnostic capabilities of the agent on this area, which also effects the efficacy of the treatment.

Therefore, using the inventive method, the dose for achieving a desired effect on a targeted area can be calculated to obtain the desired degree of the therapeutic effect, since in case a therapeutic agent is used, not any dosis of this agent or drug leads to the desired beneficial effect for the agent and too high amounts could even be detrimental or toxic.

Since it is possible according to the present invention to also take the patient dependency into account, additionally to the agent dependency, the therapeutic efficiency can be simulated and predicted and it can be determined how and why some specific agent or drug is effective for some patient group and inefficient for others.

According to the invention, it can be simulated and examined whether or not a specific drug or agent is efficient, if it is used or administered in combination with another drug or agent. Using the above described method for simulating the distribution of an agent in tissue for two or more administered agents or drugs, it can be determined whether there is any interaction between the respective administered agents or substances, directly or in interaction with the subject or patient considering e.g. metabolization, and whether or not the combined administering of these substances effects the efficiency of each single administered agent or not.

In case a diagnostic agent is administered, like chelates of Gadolinium, which has a well-known diagnostic effect for the recognition of a brain tumor, not any dosis is useful for identifying the tumor. According to the present invention, the information regarding the desired diagnostic effect can be used to calculate by way of simulation the way and dose the agent is administered and this can be made available for a specific patient before the treatment procedure starts or to track therapeutic effects during a treatment.

Preferably, the therapeutical and or diagnostic agents according to the invention are administered by convection-enhanced delivery methods, which are also referred to as high-flow microinfusion or diffusional delivery methods, which are also referred to as low-flow microinfusion. The therapeutic or diagnostic substance or portions thereof can thus be applied locally using a pressure-driven infusion delivery or a diffusion mediated delivery. It is furthermore possible to orally administer a substance or an agent or in general to apply an agent systemically using the bloodstream and/or other body fluids of a subject or patient as the transport mechanism towards the volume of interest. Furthermore, the inventive method for simulating the distribution can also be applied on the respective delivery methods to examine the effect of the administered substance on the respective transport mechanism, such as an intravenous administration.

It is also possible to perform a topical administration so that the parts of a body or patient involved in the topical delivery are considered as the volume of interest for simulating the distribution of the agent.

Preferably the choice of the substance property to be displayed depends on the expecting effect or response on the tissue. This effect may be determined by a single property or by a combination of different properties. A pre-determined effect can for example be a metabolic change of the tissue or tumor growth changes. Therefore the property of the substance to be displayed could be for those cases metabolic/reaction activity or local substance concentration, respectively.

Thus, the present invention preferably focuses on the therapeutic and/or diagnostic effects triggered by the activation or alteration of a body or body structure or tissue by the effect of the administered agent or substance.

The invention concerns also a computer program which, when loaded or running on a computer, performs the method as described herein. Furthermore, the invention relates to program storage medium or computer program product comprising such a computer program.

An apparatus according to the invention comprises a computer, a display unit connected to the computer and a computer program as mentioned above, which uses the patient-specific information, information about the administration of and information specific to a therapeutic and/or diagnostic substance and combines these pieces of information to calculate and simulate the distribution of the substance or agent in the patient or in a specific subject or tissue to generate an image which can be output on the display to display regions in which the therapeutic and/or diagnostic substance has an assumed effect on a volume of interest within the subject or patient. The computer or processor can be programmed to process information supplied by the planner to develop a graphical representation of the volume of interest or anatomical feature undergoing treatment, to develop a visual representation of the propagation of the agent (contour lines).

According to the present invention signals can be processed to generate a visual display of the targeted tissue. Efficiency contour lines representative of the degree of the effect can be superimposed over the volume of interest or anatomical feature displayed so as to indicate the effect of the administered substance on the patient.

The present invention provides a convenient way for a planner to examine which effect an administered substance on a targeted tissue has accounting for tissue-specific biological factors and concentration.

The above and other objects, features and advantages of the invention will become apparent from a consideration of the following detailed description presented in connection with the accompanying drawings in which:
FIG. 1 is a flow chart of the method of a preferred embodiment of the present invention which explains the steps of simulating the effect of an administered substance;
FIG. 2 is a modified flowchart of the present invention showing additional steps in an alternative embodiment; and
FIG. 3 is a block diagram of the apparatus according to a preferred embodiment of the present invention, including a graphical display, a general purpose computer, a keyboard and a database for entering data.

The method for displaying the regions in which an administered substance has an assumed effect includes the combination of patient-specific and agent-specific information to calculate and then display the distribution of the effect e.g. by effect contour lines indicating the degree of the effect. As understood by those skilled in the art, these contour lines can be displayed superimposed upon an image of patient tissue. The displayed combination is referred to as an effect distribution. Typically, the image is a cross section of the patient's tissue which is to receive the treatment or which has to be diagnosed, but it can also be a three dimensional image, with three dimensional effect contour lines.

The patient-specific and agent-specific information together with the administration information, which are combined for simulating or planning, can be a physical distribution of the agent or substance calculated from the administration information, e.g. infusion sites and rates, and an agent or substance activity factor. The physical distribution can be a mathematical description of the patient's tissue and the distribution of the agent therein, which is to be subjected to therapy or diagnosis, and is obtained typically through a computer calculation. Once calculated, the physical distribution is stored for retrieval when needed to calculate the effect distribution.

An agent activity factor can be the information about metabolization of the agent in the subject of interaction with another agent, which is combined with the physical distribution to obtain the effect distribution. The agent activity factor itself can be a result which is derived from a combination of considering metabolization, loss, influx, efflux, dose or weighting factors as will be explained. Consequently, the effect distribution displayed as contour lines on a cross section of the patient's tissue is the predicted effect of the administered agent, e.g. the treatment based on the calculated physical distribution and the preselected agent activity factor(s). The method can be performed a) by calculating the physical distribution, b) determining the effect distribution by combining the physical distribution and the agent activity factor(s), and c) displaying the effect distribution as contour lines superimposed upon a patient's tissue.

The agent activity factor, or more accurately the factors which are used to derive the agent activity factor, can be obtained by in-vitro experiments and/or histological studies or imaging techniques.

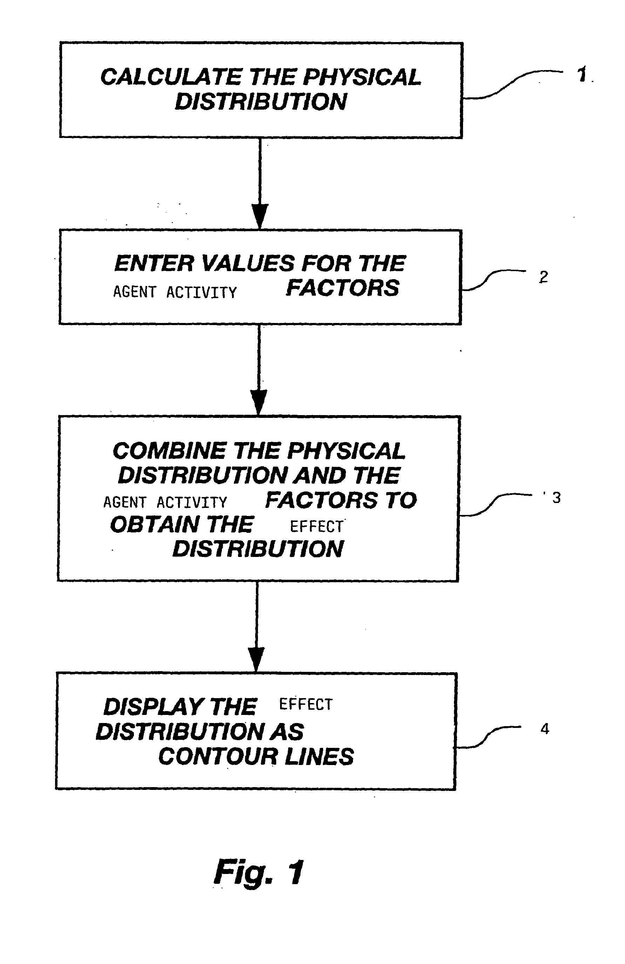

According to a preferred embodiment of the present invention the method can adjust numerous agent activity factors which are combined together to reach a final agent activity factor. The preferred embodiment of the present invention is made clear from a detailed description of the following method as also shown in FIG. 1.

The first step 1 of the method is to obtain the physical distribution, as known from the prior art. Again, the physical distribution only needs to be calculated once and can be stored for retrieval in later calculations. Therefore, the physical distribution might have been calculated previous to application of the remaining steps.

The second step 2 of the method is to enter e.g. on a computer display the values for the agent activity factors, as for example the metabolization, loss, influx, efflux, dose or weighting factors. These agent activity factors can be adjusted using an automated or manual method.

The automated method is to input the agent activity factors from a database.

The manual method of entering agent activity factors is simply to use a computer keyboard to modify the values. The values can be modified by moving a cursor using any method known to those skilled in the art such as with a mouse or moving between fields by tapping on a movement key using e.g. a keyboard. According to the preferred embodiment of the present invention the agent activity factors are adjustable e.g. to enter the metabolization parameters for respective different agents which are administered in combination or to consider the interaction of different substances when administered.

Continuing with the steps leading to the improved planning process, the third step 3 comprises using the agent activity factor(s) and the previously calculated physical distribution to generate the effect distribution.

In step 4 the graphical representation of the effect distribution is displayed.

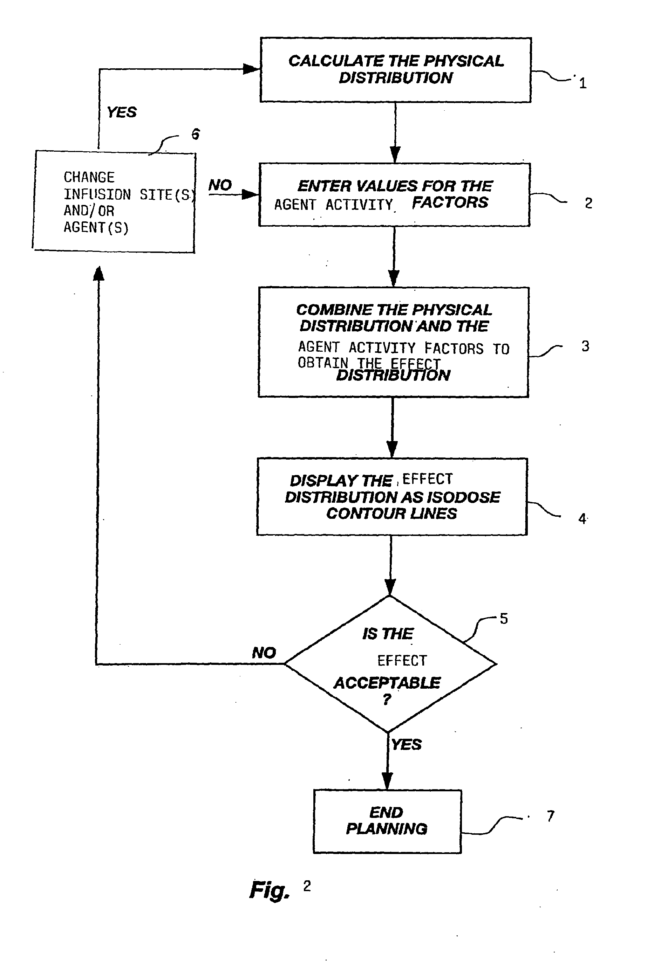

FIG. 2 shows a flow chart which adds additional steps to the method of the preferred embodiment. In this alternative embodiment, after generating the image in step 4, the planner may decide in the step 5 that the ways of administering, as e.g. infusion sites, delivery rates and/or the administered agent(s) need to be modified to change the physical distribution of the agent in the subject calculated in step 1 to obtain a more advantageous or acceptable effect distribution.

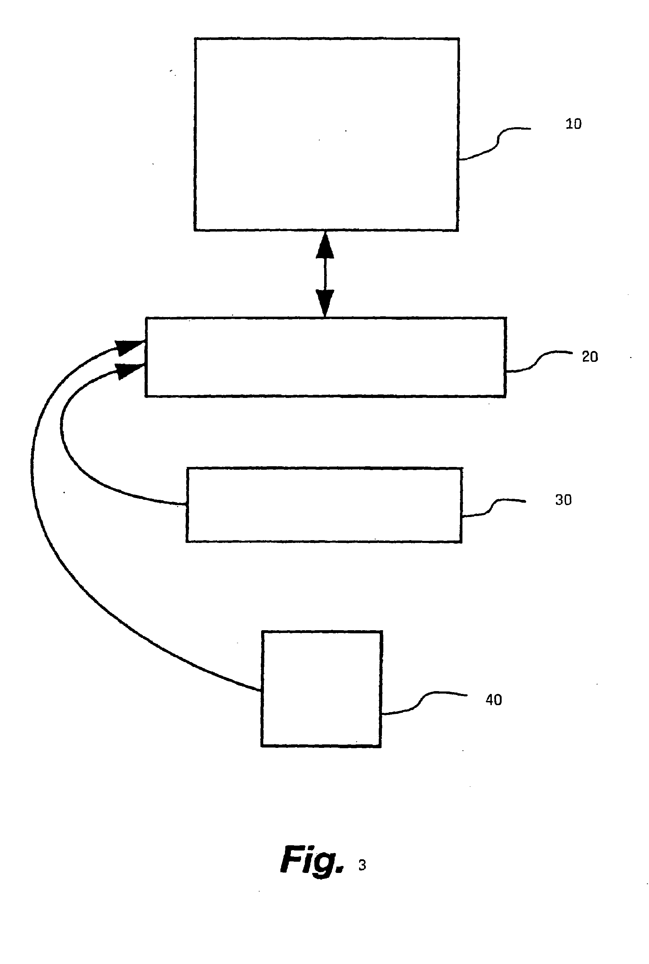

Aside from the method of the present invention, the system for executing the steps of the preferred embodiment includes according to an embodiment the components shown in block diagram form in FIG. 3:
(1) a graphical display 10 for displaying the effect distribution or "efficiency window", preferably superimposed over an image of the patient's tissue;
(2) a means for carrying out the computations necessary for obtaining a physical distribution, the final agent activity factor(s) and the effect distribution. This means is typically a general purpose computer 20; and
(3) a means for entering the agent activity factors to be displayed and used in the effect distribution calculations, such as a computer keyboard 30 or a databank 40.

## Claims

1. Method for simulating the distribution of at least an agent in a subject, wherein the at least one agent is administered to the subject, using subject-specific information, information about the administration of the at least one agent and agent-specific information, which are combined to calculate the effect of the at least one agent and regions which can be only in a volume of interest, where the agent has an effect in the subject.

2. Method according to claim 1, wherein the subject-specific information is information about physiology, anatomy, vascular permeability, hydraulic conductivity and/or diffusivity.

3. Method according to one of the preceding claims, wherein the subject-specific information is derived from MR scans, especially MRI diffusion scans, MRI dynamic contrast enhanced scans, MRI T1w scans, MRI T2w scans, MRI spectroscopy scans, CT perfusion scans, PET scans, SPECT scans, molecular imaging, CT, X-ray, ultrasound and/or electrography.

4. Method according to one of the preceding claims, wherein the agent-specific information is information about the metabolization or interaction of the agent with the subject, e.g. a tissue and/or another agent.

5. Method according to one of the preceding claims, wherein the agent-specific information used to calculate the effect distribution is obtained by in-vitro experiments and/or histological studies or imaging techniques.

6. Method according to one of the preceding claims, wherein the information about the administration of the at least one agent is an information about the way of application, e.g. local, systemical or topical application, and parameters thereof, e.g. infusion or injection sites, delivery rate(s), distribution mechanism, loss, influx and/or efflux of the agent(s) in the subject.

7. Method according to any of the preceding claims, wherein the effect of the agent is at least one of the following: binding, necrosis, swelling, uptake, metabolic alterations, genetic alterations, physical alterations, chemical alterations, electrical alterations, morphologic alterations, mutations, physiological alterations.

8. Method according to one of the preceding claims, wherein generalized data is assigned to portions of the volume of interest based on the subject-specific information and based on knowledge about the anatomic or physiological segments occurring therein.

9. A method of any of the foregoing claims in which a window of effectiveness is displayed, i.e. regions in which information regarding to effects of the therapeutic and/or diagnostic substances is pre-determined, e.g. toxicity level at the volume of interest.

10. A method of any of the foregoing claims in which the therapeutic and/or diagnostic substance or portions thereof is to be applied locally using a pressure-driven infusion delivery or a diffusion mediated delivery.

11. A method of any of the foregoing claims in which the therapeutic and/or diagnostic substance or portions thereof is to be applied systemically using the bloods stream and/or other body fluids of a subject as the volume of interest or as a transport mechanism towards the volume of interest.

12. A method of any of the foregoing claims in which the therapeutic and/or diagnostic substance or portions thereof is to be applied by topical delivery as the volume of interest or as a transport mechanism towards the volume of interest.

13. A method of any of the foregoing claims in which a variety of therapeutic and/or diagnostic substances are applied subsequently or in parallel, and in which the interactions of the various substances with the subject is taken into account to compute and display the regions in which the therapeutic and/or diagnostic substances have a pre-determined effect on a volume of interest within such subject.

14. A method of any of the foregoing claims in which one or more therapeutic and/or diagnostic substances is administered subsequently or in parallel with energy, and in which the interactions of the various substances and the energy with one another and with the subject is taken into account to compute and display the regions in which the therapeutic and/or diagnostic substances and the energy have a pre-determined effect on a volume of interest within such subject.

15. A method of any of the previous claims in which a property of an administered substance is displayed under consideration of the pre-determined effect or under consideration of one or more properties characterizing the pre-determined effect of the infused substance on the target volume.

16. Computer program, which, when loaded or running on a computer, performs the method of one of the preceding claims.

17. Program storage medium or computer program product comprising the program according to the previous claim.

18. Apparatus for simulating the distribution of an agent in tissue of a subject comprising a computer (20) and an input unit (30) or database (40) connected to the computer (30) for inputting at least one agent activity factor and the physical distribution of the agent in the subject to calculate a simulated distribution by a method as claimed in one of claims 1 to 15 and an output device (10) to display the calculated distribution.
